# NEUE EUROPÄISCHE PATENTSCHRIFT

(11) **EP 0 996 501 B2**
(45) Veröffentlichungstag und Bekanntmachung des Hinweises auf die Entscheidung über den Einspruch: **12.12.2007**
(45) Hinweis auf die Patenterteilung: 07.01.2004
(21) Anmeldenummer: 98932130.2
(22) Anmeldetag: 05.06.1998
(51) Int. Cl.: B01J 29/90, B01J 38/14, C07D 301/12, C07B 41/00

(54) **VERFAHREN ZUR REGENERIERUNG EINES ZEOLITH-KATALYSATORS**
METHOD FOR REGENERATING A ZEOLITIC CATALYST
PROCEDE POUR LA REGENERATION D'UN CATALYSEUR ZEOLITIQUE

(30) Priorität: 06.06.1997 DE 19723949
(43) Veröffentlichungstag der Anmeldung: 03.05.2000
(73) Patentinhaber: BASF Aktiengesellschaft, 67056 Ludwigshafen (DE)
(72) Erfinder: GROSCH, Georg, Heinrich, D-67098 Bad Dürkheim (DE); MÜLLER, Ulrich, D-67435 Neustadt (DE); WALCH, Andreas, D-69120 Heidelberg (DE); RIEBER, Norbert, D-68259 Mannheim (DE); HARDER, Wolfgang, D-69469 Weinheim (DE)
(74) Vertreter: Isenbruck, Günter
(86) Internationale Anmeldenummer: PCT/EP1998/003396
(87) Internationale Veröffentlichungsnummer: WO 1998/055228

(56) Entgegenhaltungen:
- EP-A- 0 604 689
- EP-A- 0 743 094
- EP-A- 0 790 075
- WO-A-94/05418
- DE-A- 4 425 672
- SU-A- 1 456 218
- US-A- 3 069 362
- US-A- 4 447 669
- US-A- 4 810 683
- PATENT ABSTRACTS OF JAPAN vol. 015, no. 306 (C-0856), 6. August 1991 & JP 03 114536 A (MITSUI TOATSU CHEM INC), 15. Mai 1991 in der Anmeldung erwähnt

## Beschreibung

Die vorliegende Erfindung betrifft ein Verfahren zur Regenerierung eines Zeolith-Katalysators, insbesondere eines Zeolith-Katalysators, der bei der Epoxidation von Olefinen mit einem Hydroperoxid, insbesondere von Propylen mit Wasserstoffperoxid eingesetzt wurde. Dabei erfolgt die Regenerierung durch Waschen mit einem Lösungsmittel und gezieltes Abbrennen der für die Deaktivierung verantwortlichen, überwiegend organischen Beläge in einer Inertgasatmosphäre, die genau definierte Mengen an Sauerstoff oder Sauerstoff-liefernden Substanzen enthält.

Bei der Durchführung von Umsetzungen in Gegenwart von Katalysatoren, insbesondere in Gegenwart von Katalysatoren, die Mikroporen aufweisen, wie z. B. Titansilicalit mit z.B. MFI-Struktur oder titanhaltigen Zeolithen mit z.B. BEA-Struktur, kann es zur Deaktivierung der Katalysatoren durch insbesondere organische Beläge kommen. Diese organischen Beläge können durch Calcinieren des Katalysators oder durch Waschen mit Lösungsmitteln zum größten Teil entfernt werden (M.G. Clerici, G. Bellussi, U. Romano, J. Catal., 129 (1991), S. 159 - 167).

Ferner beschreibt die EP-A 0 743 094 die Regenerierung eines Titan-enthaltenden Molekularsiebs, das in der Katalyse einer Oxidationsreaktion, wie z. B. der Epoxidation eines Olefins mit Wasserstoffperoxid oder einer anderen aktiven Sauerstoff-Verbindung verwendet wurde. Gemäß dieser Druckschrift erfolgt die dort beschriebene Regenerierung der deaktivierten Katalysatoren durch Abbrennen mittels Calcinieren der darauf befindlichen organischen Beläge mit molekularem Sauerstoff, wobei eine Calcinierungstemperatur von mehr als 150 °C und weniger als 400 °C verwendet wird.

Ferner beschreibt die JP-A 0 31 14 536 die Regenerierung eines Ti-Silicalitkatalysators für die Epoxidation durch Abbrennen der Beläge bei Temperaturen zwischen 400 °C und 500 °C oder durch Waschen der Katalysatoren bei Temperaturen, die oberhalb der Temperatur der Epoxidierung liegen. Als Lösungsmittel werden dort Wasser, Alkohole, Ketone, aliphatische und aromatische Kohlenwasserstoffe, Halogen-enthaltende Kohlenwasserstoffe, Ester, Nitrile oder Säuren angegeben.

Auch die DE-A 44 25 672 erwähnt die Regenerierung eines für die Epoxidation, insbesondere von Propylen, verwendeten Katalysators durch Abbrennen desselben in einer Sauerstoff-enthaltenden Atmosphäre bei erhöhten Temperaturen.

Ferner offenbart die EP-A 0 604 689 ein Verfahren zur Regenerierung von Zeolithkatalysatoren, wobei der Katalysaor mit einem Gasstrom bei einer Temperatur zwischen 400 und 600°C in Kontakt gebracht wird. Vorzugsweise enthält der Gasstrom zwischen 0,1 und 8 Vol.-% Sauerstoff und weniger als 0,2 Vol.-% Feuchtigkeit.

Die Regenerierungsverfahren gemäß des Standes der Technik besitzen jedoch einige für die Praxis unerwünschte Aspekte, insbesondere sofern Mikroporen enthaltende Katalysatoren, wie z. B. die bei der Epoxidierung insbesondere verwendeten Titansilicalite regeneriert werden sollen.

Einige der für Epoxidierungen bevorzugt eingesetzten Katalysatoren, wie z. B. ein Titansilicalit mit MFI-Struktur bzw. ein Titansilicalit mit BEA-Struktur besitzen Mikroporen mit Durchmessern im Bereich von ungefähr 0,5 bis ungefähr 0,6 nm bzw. ungefähr 0,6 bis ungefähr 0,7 nm. In beiden Fällen ist es jedoch unmöglich oligomere oder sogar polymere Nebenprodukte der durch diese Katalysatoren katalysierten Umsetzungen, insbesondere der Epoxidation, allein durch Waschen mit Lösungsmitteln bei höheren Temperaturen vollständig zu entfernen.

Das oben Gesagte trifft insbesondere für Mikroporen aufweisende Katalysatoren zu, ist jedoch - in Abhängigkeit vom Molekulargewicht bzw. den Dimensionen der sich während der Umsetzung bildenden oligomeren oder polymeren Nebenprodukte - auch für Katalysatoren mit Meso- und/oder Makroporen zutreffend.

Will man diese organischen Beläge aber vollständig entfernen, so ist dies nur durch Abbrennen derselben mit Sauerstoff oder mit Sauerstoff-liefernden Substanzen möglich. Die Regenerierung eines hochselektiven Zeolith-Katalysators mit seiner speziellen Struktur durch Abbrennen bei erhöhten Temperaturen ist jedoch schwierig, da eine vollständige oder lokale Überhitzung des Katalysators zu Selektivitätseinbußen als Folge der bei derartigen Überhitzungen auftretenden teilweisen oder in Extremfällen vollständigen Zerstörung der Zeolith-Katalysatoren imanenten Struktur führen kann. Führt man - um eine derartige Überhitzung zu vermeiden - das Abbrennen bei Temperaturen unter 400 °C durch, so ist bei kürzeren Calcinierungszeiten eine vollständige Entfernung der Beläge nicht gegeben. Eine vollständige Entfernung der Beläge durch sehr langes Calcinieren bei Temperaturen unter 400 °C ist jedoch wirtschaftlich uninteressant.

Somit liegt eine Aufgabe der vorliegenden Erfindung in der Bereitstellung eines Verfahrens zur Regenerierung eines Zeolith-Katalysators bei höheren Temperaturen, mit dem eine vollständige Entfernung der organischen Beläge auch bei kürzeren Calcinierungszeiten gewährleistet ist. Die Calcinierung sollte dabei so kontrolliert ablaufen, daß eine örtliche Überhitzung und damit eine irreversible Schädigung des Katalysators, die zu einem Selektivitätsverlust, zu einer verstärkten Bildung von Nebenprodukten und damit zu einer deutlich schnelleren Deaktivierung des regenerierten Katalysators beim erneuten Einsatz führt, verhindert wird.

Diese Aufgabe wird durch das erfindungsgemäße Verfahren gelöst.

Somit betrifft die vorliegende Erfindung ein Verfahren zur Regenerierung eines Zeolith-Katalysators, das die folgenden Stufen (I) und (II) umfaßt:
- (I): Aufheizen eines zumindest teilweise durch meist organische Beläge deaktivierten Katalysators auf eine Temperatur im Bereich 250 °C bis 600 °C in einer Atmosphäre, die weniger als 0,2 Vol.-% Sauerstoff enthält, und
- (II): Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von 0,1 bis 4 Vol.-% aufweist,
wobei dieser Gasstrom einen höheren Gehalt an Sauerstoff enthält als die Atmosphäre in Stufe (I),
wobei der Zeolith-Katalysator vor der Regenerierung bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren eingesetzt wurde,
und wobei der zumindest teilweise deaktivierte Katalysator vor dem Aufheizen gemäß Stufe (I) mit einem Lösungsmittel gewaschen wird, das ausgewählt wird aus der Gruppe bestehend aus Wasser, einem Alkohol, einem Aldehyd, einem Keton, einem Ether, einer Säure, einem Ester, einem Nitril, einem Kohlenwasserstoff sowie einem Gemisch aus zwei oder mehr davon, und das schon bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren als Lösungsmittel fungiert, und das Waschen so durchgeführt wird, dass die jeweils am Katalysator anhaftenden Wertprodukte von diesem entfernt werden können, und Temperatur und Druck nicht so hoch gewählt werden, dass die meist organischen Beläge entfernt werden.

Vorzugsweise umfaßt das erfindungsgemäße Verfahren eine weitere Stufe (III):
- (III): Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von mehr als 4 bis 100 Vol.-% aufweist.

Bezüglich der im Rahmen des vorliegenden Verfahrens regenerierten Zeolith-Katalysatoren existieren keine besonderen Beschränkungen bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren

Zeolithe sind bekanntermaßen kristalline Alumosilicate mit geordneten Kanalund Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolite Structure Types", Elsevier, 4. Aufl., London 1996.

Es sind nun auch Zeolithe bekannt, die kein Aluminium enthalten und bei denen im Silicatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beschrieben, beispielsweise in der EP-A 0 311 983 oder EP-A 405 978. Außer Silicium und Titan können solche Materialien auch zusätzliche Elemente wie z. B. Aluminium, Zirkonium, Zinn, Eisen, Kobalt, Nickel, Gallium, Bor oder geringe Menge an Fluor enthalten. In dem mit dem erfindungsgemäßen Verfahren vorzugsweise regenerierten Zeolith-Katalysatoren kann das Titan des Zeoliths teilweise oder vollständig durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Vorzugsweise werden Ti-, V-,Cr-, Nb-, Zr-Zeolithe, weiter bevorzugt Ti-Zeolithe und insbesondere Ti-Zeolithe, wie sie insbesondere zur Epoxidierung von Olefinen eingesetzt werden, mit dem erfindungsgemäßen Verfahren regeneriert.

Dabei sind im einzelnen Ti-, V-, Cr-, Nb- oder Zr-Zeolithe mit Pentasil-Zeolith-Struktur, insbesondere die Typen mit röntgenografischer Zuordnung zur BEA-, MOR-, TON-, MTW-, FER-, MFI-, MEL-, CHA-, ERI-, RHO-GIS-, BOG-, NON-, EMT-, HEU-, KFI-, FAU-, DDR-, MTT-, RUT-, LTL-, MAZ-, GME-, NES-, OFF-, SGT-, EUO-, MFS-, MCM-22- oder MFI/MEL-Mischstruktur sowie ITQ-4 zu nennen, wobei wiederum die mit MFI-Struktur, BEA-Struktur, MEL-Struktur, ITQ-4 bzw. MFI/MEL-Mischstruktur als besonders bevorzugt anzusehen sind. Zeolithe dieses Typs sind beispielsweise in der oben erwähnten Literaturstelle von W. M. Meier et al. beschrieben.

Als besonders bevorzugte Katalysatoren sind im einzelnen die Ti-enthaltenden Zeolith-Katalysatoren, die im allgemeinen als "TS-1", "TS-2", "TS-3", "TS-48", "TS-12" bezeichnet werden, sowie Ti-Zeolithe mit einer zu Zeolith-β isomorphen Gerüststruktur zu nennen.

Weitere im Rahmen des Verfahrens der vorliegenden Erfindung regenerierbare Zeolith-Katalysatoren sind u. a. in US-A 5,430,000 und WO 94/29408 beschrieben, deren Inhalt diesbezüglich unter Bezugnahme in die vorliegende Anmeldung einbezogen wird.

Als weitere titanhaltige Zeolithe sind solche mit der Struktur des ZSM-48, ZSM-12, Ferrierit oder β-Zeolith und des Mordenits zu nennen.

Selbstverständlich können auch Gemische aus zwei oder mehr, insbesondere der oben genannten Katalysatoren im Rahmen des erfindungsgemäßen Verfahrens regeneriert werden.

Auch bezüglich der Porengrößen bzw. der Porengrößenverteilung der erfindungsgemäß regenerierten Zeolith-Katalysatoren existieren keine besonderen Beschränkungen. So können im Rahmen des erfindungsgemäßen Verfahrens Katalysatoren regeneriert werden, die Mikroporen, Mesoporen oder sogar Makroporen, wie z.B. Ti-haltige SiO₂-Oxide mit Makroporen aufweisen. Besonders vorteilhaft läßt sich das erfindungsgemäße Verfahren zur Regenerierung von Mikroporen enthaltenden Katalysatoren einsetzen. Dies umfaßt Katalysatoren, die ausschließlich Mikroporen enthalten, sowie solche, die Mikro- und Mesoporen oder Mikro- und Makroporen oder Mikro-, Mesound Makroporen aufweisen. Der Begriff "*Mikroporen*", wie er im Rahmen der vorliegenden Anmeldung verwendet wird, beschreibt Poren mit einem Durchmesser von 2 nm oder darunter Der Begriff "Makroporen" bezeichnet Poren mit einem Durchmesser von größer ungefähr 50 nm und der Begriff "Mesoporen" bezeichnet Poren mit einem Durchmesser von > 2 nm bis ca. 50 nm, jeweils entsprechend der Definition gemäß "Pure Appl. Chem. 45, S. 71 ff, insbesondere S. 79 (1976).

Ferner lassen sich mittels des erfindungsgemäßen Verfahrens folgende Zeolith-Katalysatoren regenerieren:

Oxidationskatalysatoren mit Zeolith-Struktur, wie sie in der DE-A 196 23 611.8 beschrieben sind, die hiermit bzgl. der darin beschriebenen Katalysatoren voll umfänglich in den Kontext der vorliegenden Anmeldung durch Bezugnahme aufgenommen wird.

Dabei handelt es sich um Oxidationskatalysatoren auf der Basis von Titanoder Vanadiumsilicaten mit Zeolith-Struktur, wobei bzgl. der Zeolith-Struktur auf die vorstehend als bevorzugt angegebenen Strukturen verwiesen wird. Diese Katalysatoren sind dadurch gekennzeichnet, daß sie durch verfestigende Formgebungsprozesse geformt worden sind.

Als verfestigende Formgebungsprozesse können im Prinzip alle Methoden zur einer entsprechenden Formung verwendet werden, wie sie bei Katalysatoren allgemein üblich sind. Bevorzugt werden Verfahren, bei denen die Formgebung durch Extrusion in üblichen Extrudern, beispielsweise zu Strängen mit einem Durchmesser von üblicherweise 1 bis 10 mm, insbesondere 2 bis 5 mm, erfolgt. Werden Bindemittel und/oder Hilfsmittel benötigt, ist der Extrusion zweckmäßigerweise ein Mischungs- oder Knetprozeß vorgeschaltet. Gegebenenfalls erfolgt nach der Extrusion noch ein Calcinierungsschritt. Die erhaltenen Stränge werden gewünschtenfalls zerkleinert, vorzugsweise zu Granulat oder Splitt mit einem Partikeldurchmesser von 0,5 bis 5 mm, insbesondere 0,5 bis 2 mm. Dieses Granulat oder dieser Splitt und auch auf anderem Wege erzeugte Katalysatorformkörper enthalten praktisch keine feinkörnigeren Anteile als solche mit 0,5 mm Mindestpartikeldurchmesser.

In einer bevorzugten Ausführungsform enthält der eingesetzte geformte Oxidationskatalysator bis zu 10 Gew.-% Bindemittel, bezogen auf die Gesamtmasse des Katalysators. Besonders bevorzugte Bindemittelgehalte sind 0,1 bis 7 Gew.-%, insbesondere 1 bis 15 Gew.-%. Als Bindemittel eignen sich im Prinzip alle für derartige Zwecke eingesetzte Verbindungen, bevorzugt werden Verbindungen, insbesondere Oxide, des Siliciums, Aluminiums, Bors, Phosphors, Zirkoniums und/oder Titans. Von besonderem Interesse als Bindemittel ist Siliciumdioxid, wobei das SiO₂ als Kieselsol oder in Form von Tetraalkoxysilanen in den Formgebungsschritt eingebracht werden kann. Auch als bindemittel verwendbar sind Oxide des Magnesiums und Berylliums sowie Tone, z.B. Montmorillonite, Kaoline, Bentonite, Halloysite, Dickite, Nacrite und Anauxite.

Als Hilfsmittel für die verfestigenden Formgebungsprozesse sind beispielsweise Verstrangungshilfsmittel für die Extrusion zu nennen, ein übliches Verstrangungshilfsmittel ist Methylcellulose. Derartige Mittel werden in der Regel in einem nachfolgenden Calcinierungsschritt vollständig verbrannt.

Typischerweise stellt man die genannten Titan- und auch Vanadiumzeolithe dadurch her, daß man eine wäßrige Mischung aus einer SiO₂-Quelle, einer Titan- bzw. Vanadium-Quelle wie Titandioxid bzw. einem entsprechenden Vanadiumoxid und einer stickstoffhaltigen organischen Base ("Schablonen-Verbindung"), z.B. Tetrapropylammoniumhydroxid, gegebenenfalls noch unter Hinzufügen von basischen Verbindungen, in einem Druckbehälter unter erhöhter Temperatur im Zeitraum mehrerer Stunden oder einiger Tage umsetzt, wobei das kristalline Produkt entsteht. Diese wird abfiltriert, gewaschen, getrocknet und zur Entfernung der organischen Stickstoffbase bei erhöhter Temperatur gebrannt. In dem so erhaltenen Pulver liegt das Titan bzw. das Vanadium zumindest teilweise innerhalb des Zeolithgerüsts in wechselnden Anteilen mit vier-, fünf- oder sechsfacher Koordination vor. Zur Verbesserung des katalytischen Verhaltens kann sich noch eine mehrmalige Waschbehandlung mit schwefelsaurer Wasserstoffperoxidlösung anschließen, worauf das Titan- bzw. Vanadiumzeolith-Pulver erneut getrocknet und gebrannt werden muß; daran kann sich eine Behandlung mit Alkalimetallverbindungen anschließen, um den Zeolith von der H-Form in die Kation-Form zu überführen. Das so hergestellt Titan- bzw. Vanadiumzeolith-Pulver wird dann im Sinne der vorliegenden Erfindung wie oben beschrieben geformt. Ferner können Oxidationskatalysatoren auf der Basis von Titan- oder Vanadiumsilicaten mit Zeolith-Struktur mit einem Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber, die ebenfalls dadurch gekennzeichnet sind, daß sie durch verfestigende Formgebungsprozesse geformt worden sind, regeneriert werden. Derartige Katalysatoren sind in der DE-A 196 23 609.6 beschrieben, die hiermit bzgl. der darin beschriebenen Katalysatoren voll umfänglich in den Kontext der vorliegenden Anmeldung durch Bezugnahme aufgenommen wird.

Bezüglich der festigenden Formgebungsprozesse, der Bindemittel sowie der Hilfsmittel und der Struktur der Oxidationskatalysatoren trifft das oben bzgl. der DE-A 196 23 611.8 Gesagte zu.

Der dort beschriebene Oxidationskatalysator weist einen Gehalt von 0,01 bis 30 Gew.-%, insbesondere 0,05 bis 15 Gew.-%, vor allem 0,01 bis 8 Gew.-%, jeweils bezogen auf die Menge der Titan- oder Vanadium-Zeolithe, der genannten Edelmetallen auf. Hierbei wird Palladium besonders bevorzugt. Die Edelmetalle können auf den Katalysator in Form geeigneter Edelmetallkomponenten, beispielsweise in Form von wasserlöslichen Salzen, vor, während oder im Anschluß an den verfestigenden Formgebungsschritt aufgebracht werden.

In vielen Fällen ist es jedoch am günstigsten, die Edelmetallkomponenten erst nach dem Formgebungsschritt auf die Katalysatorformkörper zu bringen, besonders dann, wenn eine Hochtemperaturbehandlung des edelmetallhaltigen Katalysators unerwünscht ist. Die Edelmetallkomponenten können insbesondere durch Ionenaustausch, Imprägnierung oder Aufsprühen auf den geformten Katalysator gebracht werden. Das Aufbringen kann mittels organischer Lösungsmittel, wäßriger ammoniakalischer Lösungen oder überkritischer Phasen wie etwa Kohlendioxid erfolgen.

Durch den Einsatz dieser vorgenannten Methoden können durchaus verschiedenartige edelmetallhaltige Katalysatoren erzeugt werden. So kann durch Aufsprühen der Edelmetallösung auf die Katalysatorformteile eine Art Schalenkatalysator erzeugt werden. Die Dicke dieser edelmetallhaltigen Schale läßt sich durch Imprägnieren deutlich vergrößern, während beim Ionenaustausch die Katalysatorpartikel weitgehend gleichmäßig über den Formkörperquerschnitt mit Edelmetall belegt werden.

Ferner können die folgenden Katalysatoren erfindungsgemäß regeneriert werden:
Ein mindestens ein poröses oxidisches Material enthaltender Formkörper, der erhältlich ist durch ein Verfahren, das die folgenden Stufen umfaßt:
   (I) Versetzen eines Gemischs enthaltend ein poröses oxidisches Material oder ein Gemisch aus zwei oder mehr davon mit einer Mischung enthaltend mindestens einen Alkohol und Wasser, und
   (II) Kneten, Verformen, Trocknen und Calcinieren des gemäß Stufe (I) versetzten Gemischs.

Die Herstellung der oben beschriebenen Formkörper ausgehend von einem porösen oxidischen Material in Pulverform beinhaltet die Bildung einer plastischen Masse, die mindestens ein poröses oxidisches Material, ein Bindemittel, eine Mischung enthaltend mindestens einen Alkohol und Wasser, gegebenenfalls eine oder mehrere organische viskositätssteigernde Substanzen und weitere aus dem Stand der Technik bekannte Zusatzstoffe enthält.

Die durch inniges Vermischen, insbesondere Kneten der obigen Komponenten erhaltene plastische Masse wird vorzugsweise durch Strangpressen oder Extrudieren verformt und der erhaltene Formkörper wird nachfolgend getrocknet und abschließend calciniert.

Bezüglich der zur Herstellung des Formkörpers verwendbaren porösen oxidischen Materialien existieren keine besonderen Beschränkungen, solange es möglich ist, ausgehend von diesen Materialien einen wie hierin beschriebenen Formkörper herzustellen.

Vorzugsweise ist das poröse oxidische Material ein Zeolith, weiter bevorzugt ein Titan-, Zirkonium-, Chrom-, Niob-, Eisen- oder Vanadium-haltiger Zeolith und insbesondere ein Titansilicalit.

Bezüglich der Zeolithe, insbesondere deren Struktur und Zusammensetzung wird wiederum auf die vorstehende Diskussion der im Rahmen der Anmeldung diskutierte, mit dem erfindungsgemäßen Verfahren zu regenerierenden Zeolithe verwiesen.

Üblicherweise stellt man die genannten Titan-, Zirkonium-, Chrom-, Niob-, Eisen- und Vanadiumzeolithe dadurch her, daß man eine wäßrige Mischung aus einer SiO₂-Quelle, einer Titan-, Zirkonium-, Chrom-, Niob-, Eisen bzw. Vanadium-Quelle, wie z.B. Titandioxid bzw. einem entsprechenden Vanadiumoxid, Zirkoniumalkoholat, Chromoxid, Nioboxid oder Eisenoxid und einer stickstoffhaltigen organischen Base als Templat ("Schablonen-Verbindung"), wie z.B. Tetrapropylammoniumhydroxid, gegebenenfalls noch unter Hinzufügen von basischen Verbindungen, in einem Druckbehälter unter erhöhter Temperatur im Zeitraum mehrerer Stunden oder einiger Tage umsetzt, wobei ein kristallines Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan, bzw. das Zirkonium, Chrom, Niob, Eisen und/oder Vanadium zumindest teilweise innerhalb des Zeolithgerüsts in wechselndem Anteil mit 4-, 5- oder 6-facher Koordination vor. Zur Verbesserung der katalytischen Verhaltens kann sich noch eine mehrmalige Waschbehandlung mit schwefelsaurer Wasserstoffperoxidlösung anschließen, worauf das Titan- bzw. Zirkonium-, Chrom-, Niob-, Eisen-, Vanadiumzeolith-Pulver erneut getrocknet und gebrannt werden muß; daran kann sich eine Behandlung mit Alkalimetallverbindungen anschließen, um den Zeolith von der H-Form in die Kation-Form zu überführen. Das so hergestellte Titan- bzw. Zirkonium-, Chrom-, Niob-, Eisen-, Vanadiumzeolith-Pulver wird dann, wie nachstehend beschrieben, zu einem Formkörper verarbeitet.

Bevorzugte Zeolithe sind Titan-, Zirkonium-, Chrom-, Niob- oder Vanadiumzeolithe, weiter bevorzugt solche mit Pentasil-Zeolith-Struktur, insbesondere die Typen mit röntgenographischer Zuordnung zur BEA-, MOR-TON-, MTW-, FER-, MFI-, MEL-, CHA-, ERI-, RHO-, GIS-, BOG-, NON-, EMT-, HEU-, KFI-, FAU-, DDR-, MTT-, RUT-, LTL-, MAZ-, GME-, NES-, OFF-, SGT-, EUO-, MFS-, MCM-22- oder MFI/MEL-Mischstruktur. Zeolithe dieses Typs sind beispielsweise in der oben angegebenen Literaturstelle von Meier und Olson beschrieben. Denkbar sind weiterhin titanhaltige Zeolithe mit der Struktur des UTD-1, CIT-1, CIT-5, ZSM-48, MCM-48, ZSM-12, Ferrierit oder β-Zeolith und des Mordenits. Derartige Zeolithe sind unter anderem in der US-A 5,430,000 und der WO 94/29408 beschrieben, deren diesbezüglicher Inhalt voll umfänglich in die vorliegende Anmeldung durch Bezugnahme aufgenommen wird.

Auch bezüglich der Porenstruktur der erfindungsgemäß zu regenerierenden Formkörper existieren keine besonderen Beschränkungen, d.h. der erfindungsgemäße Formkörper kann Mikroporen, Mesoporen, Makroporen, Mikro- und Mesoporen, Mikro- und Makroporen oder Mikro-, Meso- und Makroporen aufweisen, wobei die Definition der Begriffe "Mesoporen" und "Makroporen" ebenfalls derjenigen in oben erwähnter Literatur gemäß Pure Appl. Chem. entspricht und Poren mit einem Durchmesser von > 2 nm bis ca. 50 nm bzw. > ungefähr 50 nm bezeichnet.

Ferner kann mittels des erfindungsgemäßen Verfahrens ein Material auf der Basis eines mesoporösen siliziumhaltigen Oxids sowie eines siliziumhaltigen Xerogels regeneriert werden.

Besonders bevorzugt sind siliziumhaltige mesoporöse Oxide, die noch Ti, V, Zr, Sn, Cr, Nb oder Fe, insbesondere Ti, V, Zr, Cr, Nb oder ein Gemisch aus zwei oder mehr davon, enthalten.

Als Bindemittel eignen sich im Prinzip alle für derartige Zwecke bislang eingesetzten Verbindungen. Bevorzugt werden Verbindungen, insbesondere Oxide des Siliziums, Aluminiums, Bors, Phosphors, Zirkoniums und/oder Titans verwendet. Von besonderem Interesse als Bindemittel ist Siliziumdioxid, wobei das SiO₂ als Kieselsol oder in Form von Tetraalkoxysilanen in den Formgebungsschritt eingebracht werden kann. Ferner sind Oxide des Magnesiums und Berylliums sowie Tone, z.B. Montmorillonite, Kaoline, Bentonite, Halloysite, Dickite, Nacrite und Anauxite als Bindemittel verwendbar.

Vorzugsweise wird als Bindemittel jedoch ein Metallsäureester oder ein Gemisch aus zwei oder mehr davon in Stufe (I) des erfindungsgemäßen Verfahrens zugesetzt. Als solche sind insbesondere Orthokieselsäureester, Tetraalkoxysilane, Tetraalkoxytitanate, Trialkoxyaluminate, Tetraalkoxyzirkonate oder ein Gemisch aus zwei oder mehr davon zu nennen.

Besonders bevorzugt werden im Rahmen der vorliegenden Erfindung jedoch Tetraalkoxysilane als Bindemittel verwendet. Im einzelnen zu nennen sind dabei Tetramethoxysilan, Tetraethoxysilan, Tetrapropoxysilan und Tetrabutoxysilan, die analogen Tetraalkoxytitan- und -zirkonium-Verbindungen sowie Trimethoxy-, Triethoxy-, Tripropoxy-, Tributoxyaluminium, wobei Tetramethoxysilan und Tetraethoxysilan besonders bevorzugt sind.

Der Formkörper enthält vorzugsweise bis zu ungefähr 80 Gew.-%, weiter bevorzugt ungefähr 1 bis ungefähr 50 Gew.-% und insbesondere ungefähr 3 bis ungefähr 30 Gew.-% Bindemittel, jeweils bezogen auf die Gesamtmasse des Formkörpers, wobei die Menge an Bindemittel sich aus dem entstehenden Metalloxid ergibt.

Der vorzugsweise verwendete Metallsäureester wird in einer solchen Menge eingesetzt, daß der daraus entstehende Metalloxid-Gehalt im Formkörper ungefähr 1 bis ungefähr 80 Gew.-%, vorzugsweise ungefähr 2 bis ungefähr 50 Gew.-% und insbesondere ungefähr 3 bis ungefähr 30 Gew.-%, jeweils bezogen auf die Gesamtmasse des Formkörpers liegt.

Wie sich aus obigem bereits ergibt, können selbstverständlich auch Gemische aus zwei oder mehr der oben genannten Bindemittel eingesetzt werden.

Essentiell für die Herstellung dieser Formkörper ist es, daß als Anteigungsmittel eine Mischung enthaltend mindestens einen Alkohol und Wassers verwendet wird. Dabei beträgt der Alkoholgehalt dieser Mischung im allgemeinen ungefähr 1 bis ungefähr 80 Gew.-%, vorzugsweise ungefähr 5 bis ungefähr 70 Gew.-% und insbesondere ungefähr 10 bis ungefähr 60 Gew.-%, jeweils bezogen auf das Gesamtgewicht der Mischung.

Vorzugsweise entspricht der verwendete Alkohol der Alkoholkomponente des als Bindemittel vorzugsweise verwendeten Metallsäureesters, wobei es jedoch auch nicht kritisch ist, einen anderen Alkohol zu verwenden.

Bezüglich der verwendbaren Alkohole bestehen keinerlei Beschränkungen, sofern sie wassermischbar sind. Es können demnach sowohl Monoalkohole mit 1 bis 4 C-Atomen und wassermischbare mehrwertige Alkohole verwendet werden. Insbesondere werden Methanol, Ethanol, Propanol sowie n-, iso-, tert.-Butanol, sowie Gemische aus zwei oder mehr davon verwendet.

Als organische viskositätssteigernde Substanz können ebenfalls alle dafür geeigneten, aus dem Stand der Technik bekannten Substanzen verwendet werden. Vorzugsweise sind dies organische, insbesondere hydrophile Polymere, wie z.B. Cellulose, Stärke, Polyacrylate, Polymethacrylate, Polyvinylalkohol, Polyvinylpyrrolidon, Polyisobuten, Polytetrahydrofuran. Diese Substanzen fördern in erster Linie die Bildung einer plastischen Masse während des Knet-, Verformungs- und Trocknungsschritts durch Verbrücken der Primärpartikel und gewährleisten darüber hinaus die mechanische Stabilität des Formkörpers beim Verformen und Trocknen. Diese Substanzen werden beim Calcinieren wieder aus dem Formkörper entfernt.

Als weitere Zusatzstoffe können Amine oder aminartige Verbindungen, wie z.B. Tetraalkylammoniumverbindungen oder Aminoalkohole, sowie carbonathaltige Substanzen, wie z. B. Calciumcarbonat, zugesetzt werden. Derartige weitere Zusatzstoffe sind in EP-A 0 389 041, EP-A 0 200 260 und in WO 95/19222 beschrieben, die diesbezüglich vollumfänglich in den Kontext der vorliegenden Anmeldung durch Bezugnahme einbezogen werden.

Statt basischer Zusatzstoffe ist es auch möglich saure Zusatzstoffe zu verwenden. Diese können unter anderem eine schnellere Reaktion des Metallsäureesters mit dem porösen oxidischen Material bewirken. Bevorzugt sind organische saure Verbindungen, die sich nach dem Verformungsschritt durch Calcinieren herausbrennen lassen. Besonders bevorzugt sind Carbonsäuren.

Selbstverständlich können auch Gemische aus zwei oder mehr der oben genannten Zusatzstoffe eingebaut werden.

Die Zugabereihenfolge der Bestandteile der das poröse oxidische Material enthaltenden Masse ist nicht kritisch. Es ist sowohl möglich, zuerst das Bindemittel zuzugeben, anschließend die organische viskositätssteigernde Substanz, ggf. den Zusatzstoff und zum Schluß die Mischung enthaltend mindestens einen Alkohol und Wassers, als auch die Reihenfolge bezüglich des Bindemittels, der organischen viskositätssteigernden Substanz und der Zusatzstoffe zu vertauschen.

Nach der Zugabe des Bindemittels zum pulverförmigen porösen Oxid, dem gegebenenfalls die organische viskositätssteigernde Substanz bereits zugegeben worden ist, wird die in der Regel noch pulverförmige Masse 10 bis 180 Minuten im Kneter oder Extruder homogenisiert. Dabei wird in der Regel bei Temperaturen im Bereich von ungefähr 10 °C bis zum Siedepunkt des Anteigungsmittel und Normaldruck oder leichtem überathmosphärischem Druck gearbeitet. Danach erfolgt die Zugabe der restlichen Bestandteile, und das so erhaltene Gemisch wird solange geknetet, bis eine verstrangbare oder extrudierfähige, plastische Masse entstanden ist.

Prinzipiell können für die Knetung und die Verformung alle herkömmlichen Knet- und Verformungsvorrichtungen bzw. Verfahren, wie sie zahlreich aus dem Stand der Technik bekannt sind und für die Herstellung von z.B. Katalysator-Formkörpern allgemein verwendet werden.

Dabei sind jedoch Verfahren bevorzugt, bei denen die Verformung durch Extrusion in üblichen Extrudern, beispielsweise zu Strängen mit einem Durchmesser von üblicherweise ungefähr 1 bis ungefähr 10 mm, insbesondere ungefähr 2 bis ungefähr 5 mm, erfolgt. Derartige Extrusionsvorrichtungen werden beispielsweise in Ullmanns Enzyklopädie der Technischen Chemie, 4. Auflage, Bd. 2, S. 295 ff., 1972 beschrieben. Neben der Verwendung eines Extruders wird ebenfalls vorzugsweise eine Strangpresse zur Verformung verwendet.

Nach Beendigung des Strangpressens oder Extrudierens werden die erhaltenen Formkörper bei im allgemeinen ungefähr 30 °C bis 140 °C (1 bis 20 h, Normaldruck) getrocknet und bei ungefähr 400 °C bis ungefähr 800 °C (3 bis 10 h, Normaldruck) calciniert.

Selbstverständlich können die erhaltenen Stränge bzw. Extrudate zerkleinert werden. Sie werden dabei vorzugsweise zu einem Granulat oder Splitt mit einem Partikeldurchmesser von 0,1 bis 5 mm, insbesondere 0,5 bis 2 mm zerkleinert.

Dieses Granulat oder dieser Splitt und auch auf anderem Wege erzeugte Formkörper enthalten praktisch keine feinkörnigeren Anteile als solche mit ungefähr 0,1 mm Mindestpartikeldurchmesser.

Im Rahmen des erfindungsgemäßen Verfahrens können sowohl Katalysatoren in Pulverform, die als Suspension verwendet werden, als auch in einem Festbett gepackte Katalysatoren in Form eines Formkörpers sowie auf Netze, wie z.B. ein Edelstahl, Kanthal oder Packungen kristallisierte Katalysatoren und Schalenkatalysatoren, bestehend aus inertem Kern aus SiO₂, α-Al₂O₃, hochcalciniertem TiO₂, Steatit und einer aktiven Kalaysatorhülle, die ein Zeolith, vorzugsweise ein Zeolith wie oben definiert, umfaßt, die regeneriert werden.

Sofern der Katalysator in Suspensionsfahrweise verwendet wurde, muß er zunächst durch einen Abtrennschritt, wie z.B. Filtration oder Zentrifugieren von der Reaktionslösung abgetrennt werden. Der so gewonnene, zumindest teilweise deaktivierte pulverförmige Katalysator kann dann der Regenerierung zugeführt werden. Die während des Regenerierungsverfahrens bei erhöhten Temperaturen durchgeführten Stufen werden bei derartigen pulverförmigen Katalysatoren vorzugsweise in Drehrohröfen durchgeführt. Bei der Regenerierung eines Katalysators, der in Suspensionsfahrweise verwendet wird, ist es besonderes bevorzugt im Rahmen einer Kopplung der Umsetzung in Suspensionsfahrweise und des erfindungsgemäßen Regenerierungsverfahrens kontinuierlich einen Teil des zumindest teilweise deaktivierten Katalysators aus der Umsetzung zu entfernen, extern mittels des erfindungsgemäßen Verfahrens zu regenerieren und den regenerierten Katalysator wieder in die Umsetzung in Suspensionsfahrweise einzuschleusen.

Neben der Regenerierung von Katalysatoren in Pulverform können mit dem erfindungsgemäßen Verfahren auch Katalysatoren als Formkörper, beispielsweise solche, die in einem Festbett gepackt sind, regeneriert werden. Bei der Regenerierung eines im Festbett gepackten Katalysators erfolgt die Regenerierung vorzugsweise in der Umsetzungsvorrichtung selbst, wobei der Katalysator dazu weder aus- noch eingebaut werden muß, so daß er keinerlei zusätzlicher mechanischer Belastung unterliegt. Bei der Regenerierung des Katalysators in der Umsetzungsvorrichtung an sich wird zunächst die Umsetzung unterbrochen, gegebenenfalls vorhandenes Umsetzungsgemisch entfernt, die Regenerierung durchgeführt und anschließend die Umsetzung fortgesetzt.

Sowohl bei der Regenerierung von pulverförmigen Katalysatoren als auch bei der Regenerierung von Katalysatoren in verformter Form verläuft die erfindungsgemäße Regenerierung im Wesentlichen identisch.

Gemäß Stufe (I) wird der Katalysator entweder in der Umsetzungsvorrichtung oder in einem externen Ofen in einer Atmosphäre, die weniger als 0,2 Vol.-% Sauerstoff enthält auf eine Temperatur im Bereich von ungefähr 250 °C bis ungefähr 600 °C, vorzugsweise ungefähr 400 °C bis 550 °C und insbesondere ungefähr 450 °C bis 500 °C aufgeheizt. Dabei wird das Aufheizen gemäß Stufe (I) vorzugsweise mit einer Aufheizrate von ungefähr 0,1 °C/min. bis ungefähr 20 °C/min., vorzugsweise ungefähr 0,3 °C/min. bis ungefähr 15 °C/min. und insbesondere 0,5 °C/min. bis 10 °C/min. durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und nicht derart ansteigt, daß es zu Schädigungen der Katalysatorstruktur kommt.

Nach dem Erreichen des für die Zersetzung der Beläge gewünschten Temperaturbereichs von ungefähr 250 °C bis ungefähr 800 °C, vorzugsweise ungefähr 350 °C bis ungefähr 600 °C und insbesondere ungefähr 400 °C bis ungefähr 600 °C kann - sofern dies erwünscht, oder beim Vorliegen einer großen Menge an organischen Belägen notwendig ist - der Katalysator gegebenenfalls weitere 1 bis 2 Stunden bei diesen Temperaturen in der oben definierten Atmosphäre belassen werden.

In Stufe (I) der Regenerierung, gegebenenfalls zusammen mit dem Belassen des Katalysators bei der angegebenen Temperatur, wird der Großteil der Beläge verkokt. Die dabei gebildeten Substanzen, wie z. B. Wasserstoff, Wasser, kohlenstoffhaltige Substanzen werden im Rahmen dieser Stufe vom Katalysator entfernt. Die im Rahmen dieser Stufe betriebene Entfernung der Beläge durch Verkoken vermindert in signifikantem Maße die während des Abbrennens des Katalysators im Rahmen der Stufen (II) und ggf. (III) des erfindungsgemäßen Verfahrens durch Beaufschlagen des Katalysators mit einem Gasstrom, der einen höheren Gehalt an Sauerstoff enthält, freiwerdende Energiemenge, so daß bereits durch das langsame Aufheizen gemäß Stufe (I) des erfindungsgemäßen Verfahrens ein wesentlicher Schritt zur Verhinderung einer lokalen Überhitzung des Katalysators erreicht wird.

Gemäß Stufe (II) des erfindungsgemäßen Verfahrens wird der Katalysator anschließend bei einer Temperatur im Bereich von ungefähr 250 °C bis ungefähr 800 °C, vorzugsweise ungefähr 350 °C bis ungefähr 600 °C mit einem Gasstrom, der einen Gehalt an einer Sauerstoff-liefernden Substanz oder an Sauerstoff oder an einem Gemisch aus zwei oder mehr davon im Bereich von ungefähr 0,1 bis ungefähr 4 Vol.-%, vorzugsweise ungefähr 0,1 bis ungefähr 3 Vol.-% , weiter bevorzugt ungefähr 0,1 bis ungefähr 2 Vol.-% aufweist, beaufschlagt.

Dabei ist die zugesetzte Menge an molekularem Sauerstoff oder Sauerstoff-liefernden Substanzen insoweit kritisch, als daß durch die innerhalb dieser Stufe freiwerdende Energiemenge, die durch den Abbrand der verkokten organischen Beläge entsteht, eine Erhöhung der Temperatur des Katalysators einhergeht, so daß die Temperatur in der Vorrichtung zur Regenerierung den gewünschten Temperaturbereich von ungefähr 250 °C bis ungefähr 800 °C, vorzugsweise ungefähr 350 °C bis ungefähr 600 °C nicht verlassen darf. Vorzugsweise wird die Menge an molekularem Sauerstoff oder Sauerstoff-liefernden Substanzen so gewählt, daß die Temperatur in der Vorrichtung sich zwischen ungefähr 400 °C und ungefähr 500 °C befindet.

Mit zunehmendem Abbrand der Beläge muß der Gehalt an molekularem Sauerstoff oder Sauerstoff-liefernden Substanzen im Inertgasstrom bis hin zu 100 Vol.-% gesteigert werden, um die zur Regenerierung erforderliche Temperatur aufrecht zu erhalten, so daß nach 'Beendigung der Stufe (II) im Rahmen der Stufe (III) der Katalysator im bereits bezüglich der Stufe (II) definierten Temperaturbereich mit einem Gasstrom beaufschlagt wird, der einen Gehalt an einer Sauerstoff-liefernden Substanz oder an Sauerstoff oder an einem Gemisch aus zwei oder mehr davon im Bereich von mehr als ungefähr 4 bis 100 Vol.-%, vorzugsweise mehr als ungefähr 3 Vol.-% bis ungefähr 20 Vol.-%, weiter bevorzugt ungefähr 2 Vol.-% bis ungefähr 20 Vol.-% aufweist.

Dabei wird in der Regel so vorgegangen, daß bei einem Absinken der Temperatur im Rahmen der Stufe (II) die Menge an Sauerstoff bzw. Sauerstoff-liefernder Substanz im zugeführten Gasstrom kontinuierlich erhöht wird.

Die Temperatur des Katalysators an sich wird durch entsprechende Steuerung des Sauerstoff-Gehalts bzw. des Gehalts an Sauerstoff-liefernden Substanzen im Gasstrom bei einer Temperatur im Bereich von ungefähr 250 °C bis ungefähr 800 °C, vorzugsweise ungefähr 350 °C bis ungefähr 600 °C, insbesondere ungefähr 400 °C bis ungefähr 600 °C gehalten.

Sinkt die Temperatur des Abgasstrom am Reaktorausgang trotz steigender Mengen an molekularem Sauerstoff oder Sauerstoff-liefernden Substanzen im Gasstrom, so ist das Abbrennen der organischen Beläge beendet. Die Dauer der Behandlung gemäß der Stufe (II) sowie ggf. der Stufe (III) beträgt im allgemeinen jeweils ungefähr 1 bis ungefähr 30, vorzugsweise ungefähr 2 bis ungefähr 20 und insbesondere ungefähr 3 bis ungefähr 10 Stunden.

Der obige Begriff "Sauerstoff-liefernde Substanzen" umfaßt alle Substanzen, die in der Lage sind, unter den angegebenen Regenerierungsbedingungen Sauerstoff abzugeben oder kohlenstoffhaltige Rückstände zu entfernen. Insbesondere zu nennen sind:

Stickoxide der Formel NₓO_{y}, wobei x und y so gewählt werden, daß sich ein neutrales Stickoxid ergibt, N₂O, N₂O-haltiger Abgasstrom aus einer Adipinsäureanlage, NO, NO₂, Ozon oder ein Gemisch aus zwei oder mehr davon. Bei Verwendung von Kohlendioxid als Sauerstoff-liefernde Substanz werden die Stufen (II) und (III) bei einer Temperatur im Bereich von 500 ° C bis 800 ° C durchgeführt.

Der zumindest teilweise deaktivierte Katalysator wird vor dem Aufheizen gemäß Stufe (I) mit einem Lösungsmittel gewaschen, um noch anhaftendes Wertprodukt zu entfernen. Dabei wird das Waschen so durchgeführt, daß zwar die jeweils am Katalysator anhaftenden Wertprodukte von diesem entfernt werden können, aber Temperatur und Druck nicht so hoch gewählt werden, daß die meist organischen Beläge ebenfalls entfernt werden. Vorzugsweise wird der Katalysator dabei mit einem geeigneten Lösungsmittel lediglich gespült.

Somit eignen sich für diesen Waschvorgang alle Lösungsmittel, in denen sich das jeweilige Umsetzungsprodukt gut löst. Derartige Lösungsmittel werden ausgewählt aus der Gruppe bestehend aus Wasser, einem Alkohol, wie z. B. Methanol, Ethanol, 1-Propanoi, 2-Propanoi, 2-Methyl-2-propanol, 1-Butanol, 2-Butanol, Allylalkohol oder Ethylenglycol, einem Aldehyd, wie z. B. Acet- oder Propionaldehyd, einem Keton, wie z.B. Aceton, 2-Butanon, 2-Methyl-3-butanon, 2-Pentanon, 3-Pentanon, 2-Methyl-4-pentanon oder Cyclohexanon, einem Ether wie z. B. Diethylether oder THF, einer Säure, wie z. B. Ameisensäure, Essigsäure oder Propionsäure, einem Ester, wie z. B. Methylformiat, Methylacetat, Ethylacetat, Butylacetat oder Ethylpropionat, einem Nitril, wie z. B. Acetonitril, einem Kohlenwasserstoff, wie z. B. Propan, 1-Buten, 2-Buten, Benzol, Toluol, Xylol, Trimethylbenzol, Dichlormethan, Chloroform, Tetrachlorkohlenstoff, 1,1-Dichlorethan, 1,2-Dichlorethan, 1,1,1-Trichlorethan, 1, 1,2-Trichlorethan, 1,1,1,2-Tetrachlorethan, Dibromethan, Allylchlorid oder Chlorbenzol, und, soweit mischbar, Gemische aus zwei oder mehr davon, verwendet.

Es werden Lösungsmittel, die schon bei der Umsetzung, also bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren unter Verwendung des zu regenerierenden Katalysators als Lösungsmittel fungieren, eingesetzt. Als solche sind beispielhaft für die Epoxidierung von Olefinen zu nennen: Wasser, Alkohole, wie z.B. Methanol, Ethanol, 1-Propanol, 2-Propanol, 2-Methyl-2-propanol, 1-Butanol, 2-Butanol, Allylalkohol oder Ethylenglycol, oder Ketone, wie z.B. Aceton, 2-Butanon, 2-Methyl-3-butanon, 2-Pentanon, 3-Pentanon, 2-Methyl-4-pentanon oder Cyclohexanon.

Die benutzte Menge an Lösungsmittel sowie die Dauer des Waschvorgangs sind nicht kritisch, sowohl Menge an Lösungsmittel als auch Dauer des Waschvorgangs sollten jedoch ausreichen, um einen Großteil des am Katalysator haftenden Wertprodukts zu entfernen. Der Waschvorgang kann bei der Temperatur der Umsetzung oder bei verglichen dazu erhöhten Temperaturen erfolgen, wobei die Temperatur jedoch nicht so hoch sein sollte, daß das zum Waschen verwendete Lösungsmittel selbst wieder mit dem zu entfernenden Wertprodukt reagiert. Sofern Temperaturen, die oberhalb der Umsetzungstemperatur liegen, verwendet werden, ist im allgemeinen ein Bereich von 5 °C bis 150 °C oberhalb der Umsetzungstemperatur, insbesondere auch bedingt durch den Siedepunkt der verwendeten Lösungsmittel, ausreichend. Der Waschvorgang kann falls erforderlich, mehrmals wiederholt werden. Der Waschvorgang kann unter Normaldruck, erhöhtem Druck oder sogar überkritischem Druck erfolgen. Bevorzugt sind Normaldruck und erhöhter Druck.

Wird ein in Suspensionsfahrweise verwendeter pulverförmiger Katalysator regeneriert, erfolgt das Waschen des abgetrennten Katalysators in einem externen Reaktor. Ist der Katalysator in Form eines Festbetts in einem Reaktor gepackt, so kann das Waschen im Umsetzungsreaktor erfolgen. Dabei wird dieser mit dem darin befindlichen zu regenerierenden Katalysator ein- oder mehrmals mit dem Lösungsmittel gespült, um das restliche Wertprodukt zu gewinnen. Anschließend wird das Lösungsmittel aus dem Reaktor entfernt.

Nach der Beendigung des Waschvorgangs wird der Katalysator im allgemeinen getrocknet. Obwohl der Trocknungsvorgang an sich nicht kritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere - falls vorhanden - den Mikroporen des Zeolith-Katalysators zu vermeiden, da auch dies zu einer Schädigung desselben führen kann. Bei Regenerierung von pulverförmigen Katalysatoren erfolgt die Trocknung wiederum extern in einer Heizvorrichtung unter Inertgasatmosphäre. Bei Katalysatoren im Festbett wird der im Reaktor befindliche Katalysator bei mäßigen Temperaturen mit einem Inertgasstrom beaufschlagt. Die Trocknung des Katalysators kann, muß aber nicht bis zur Vollständigkeit durchgeführt werden. Bei pulverförmigen Katalysatoren wird in der Regel so weit getrocknet, bis das Pulver rieselfähig ist. Auch bei Katalysatoren, die in einem Festbett eingebaut sind, ist eine vollständige Trocknung in der Regel nicht nötig.

Nach der Regenerierung kann der Katalysator mit basischen und/oder silylierenden Verbindungen zur Entfernung von sauren Zentren behandelt werden. Als derartige Verbindungen eignen sich dort insbesondere verdünnte wäßrige Lösungen von Alkali- oder Erdalkalihydroxiden, -carbonaten, -hydroxycarbonaten; Li-, K-, Na-Acetate und -Phosphate; sowie als silylierende Verbindungen silylierende Ester, wie z.B. Tetraalkoxysilane, Tetraalkoxymonoalkylsilane sowie Hexamethylendisilane.

In einer weiteren Ausführungsform des erfindungsgemäßen Verfahrens wird in einer zusätzlichen Stufe (IV) der in Stufe (III) erhaltene regenerierte Katalysator in einem Inertgasstrom abgekühlt. Dieser Inertgasstrom kann bis zu 20 Vol.-%, vorzugsweise ungefähr 0,5 bis ungefähr 20 Vol.-% eines Flüssigkeitsdampfes, ausgewählt aus der Gruppe bestehend aus Wasser, einem Alkohol, einem Aldehyd, einem Keton, einem Ether, Säure, einem Ester, einem Nitril, einem Kohlenwasserstoff, wie oben bezüglich des Waschens des Katalysators beschrieben, und einem Gemisch aus zwei oder mehr davon, enthalten. Vorzugsweise werden Wasser, Alkohol, oder ein Gemisch aus zwei oder mehr davon als Flüssigkeitsdampf verwendet.

Bezüglich der vorzugsweise verwendbaren Alkohole, Aldehyde, Ketone, Ether, Säuren, Ester, Nitrile oder Kohlenwasserstoffe wird auf die entsprechende Diskussion der im Rahmen des Waschvorgangs im erfindungsgemäßen Verfahren verwendbaren Lösungsmittel verwiesen.

Dabei ist es auch bei dem Abkühlen gemäß Stufe (IV) wichtig, daß langsam abgekühlt wird, da ein zu schnelles Abkühlen ("Abschrecken") die mechanische Festigkeit des Katalysators negativ beeinflussen kann. Ebenso kann die Mechanik des Katalysators durch schnelles Spülen der regenerierten, trockenen Katalysatorformkörper beim Wiederanfahren des Reaktors für die weitere Umsetzung negativ beeinflußt werden. Aus diesem Grund empfiehlt es sich, während der Abkühlphase den oben definierten Flüssigkeitsdampf zuzusetzen. Weiter bevorzugt wird dieser jedoch erst unterhalb einer sogenannten Schwellentemperatur zugesetzt, die durch die Siedetemperatur der für den Dampf verwendeten Flüssigkeit definiert wird. Die Schwellentemperatur liegt dabei in der Regel unterhalb von ungefähr 250 °C, vorzugsweise unterhalb von ungefähr 200 °C und insbesondere unterhalb von ungefähr 150 °C.

Nach dem Abkühlen des Reaktors und des darin befindlichen regenerierten Katalysators auf die Umsetzungstemperatur wird der Reaktor mit dem Umsetzungsgemisch befüllt und die Umsetzung fortgesetzt. Das erfindungsgemäße Verfahren wird zur Regenerierung von Zeolith-Katalysatoren, die bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren, also für Oxidationsreaktionen eingesetzt werden, verwendet.

### BEISPIELE

### Beispiel 1

In einem Vierhalskolben (4 l Inhalt) wurden 910 g Tetraethylorthosilicat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Anschließend versetzte man mit 1600 g einer 20 gew.-%igen Tetrapropylammoniumhydroxid-Lösung (Alkaligehalt < 10 ppm) und rührte noch eine Stunde nach. Bei 90 °C bis 100 °C wurde das aus der Hydrolyse gebildete Alkoholgemisch (ca. 900 g) abdestilliert. Man füllte mit 3 1 Wasser auf und gab das mittlerweile leicht opaque Sol in einen 5 1 fassenden Rührautoklaven aus Edelstahl.

Mit einer Heizrate von 3 °C/min wurde der verschlossene Autoklav (Ankerrührer, 200 U/min) auf eine Reaktionstemperatur von 175 °C gebracht. Nach 92 Stunden war die Reaktion beendet. Das erkaltete Reaktionsgemisch (weiße Suspension) wurde abzentrifugiert und mehrfach mit Wasser neutral gewaschen. Der erhaltene Feststoff wurde bei 110 °C innerhalb von 24 Stunden getrocknet (Auswaage: 298 g).

Anschließend wurde unter Luft bei 550 °C in 5 Stunden das im Zeolithen verbliebene Templat abgebrannt (Calcinierungsverlust: 14 Gew.-%).

Das reinweiße Produkt hatte nach naßchemischer Analyse eine Ti-Gehalt von 1,5 Gew.-% und einen Gehalt an Restalkali unterhalb 100 ppm. Die Ausbeute auf eingesetztes SiO₂ betrug 97 %. Die Kristallite hatten eine Größe von 0,05 bis 0,25 µm und das Produkt zeigte in IR eine typische Bande bei ca. 960 cm⁻¹.

### Beispiel 2

530 g Titansilicalit-Pulver, synthetisiert gemäß Beispiel 1, wurden mit 13,25 g Kieselsol (Ludox AS-40), 26,5 g Walocel (Methylcellulose) und 354 ml Wasser 2 h lang im Kneter verknetet. Die verdichtete Masse wurde dann in einer Strangpresse zu 2 mm-Strängen verformt. Die erhaltenen Stränge wurden bei 110 °C 16 h lang getrocknet und dann bei 500 °C 5 h lang calciniert.
100 g der so erhaltenen Formkörper wurden zu Splitt (Partikelgröße 1-2 mm) verarbeitet und als Katalysator in der Epoxidation von Propylen mit Wasserstoffperoxid verwendet.

### Beispiel 3

Durch eine Reaktorkaskade von zwei Reaktoren mit je 190 ml Reaktionsvolumen, gefüllt mit je 10 g Katalysator gemäß Beispiel 2, wurden Flüsse von 27,5 g/h Wasserstoffperoxid (20 Gew.-%), 65 g/h Methanol und 13,7 g/h Propen bei 40 °C Reaktionstemperatur und 20 bar Reaktionsdruck durchgeleitet. Nach Verlassen des zweiten Reaktors wurde die Reaktionsmischung in einen Sambay-Verdampfer gegen Atmosphärendurck entspannt. Die abgetrennten Leichtsieder wurden on-line in einem Gaschromatographen analysiert. Der flüssige-Reaktionsaustrag wurde gesammelt, gewogen und ebenfalls gaschromatographisch analysiert.
Während der gesamten Laufzeit sank der Wasserstoffperoxid-Umsatz von ursprünglich 98 % und erreichte nach 250 h einen Wert von ungefähr 60 % . Die Selektivität von Propylenoxid bzgl. Wasserstoffperoxid betrug über die Laufzeit 95 % .

### Beispiel 4

Der deaktivierte Katalysator aus Beispiel 3 wurde in ein Quarzglasrohr eingebaut. Der deaktivierte Katalysator wurde daraufhin in einem Röhrenofen bei einer Heizrate von 4 °C/min bei einem Gasstrom aus 20 l Stickstoff pro Stunde auf 500 °C erhitzt. Danach wurden in den nächsten zwei Stunden der Sauerstoffgehalt im Inertgas auf 9 Vol.-% gesteigert und dort gehalten. Anschließend wurde in den nächsten 14 h der Sauerstoffgehalt auf 18 Vol.-% gesteigert und dort gehalten. Danach wurde der regenerierte Katalysator unter Inertgas abgekühlt, ausgebaut und wieder zur Epoxidation verwendet.

### Beispiel 5

Durch eine Reaktorkaskade von zwei Reaktoren mit je 190 ml Reaktionsvolumen, gefüllt mit je 10 g regeneriertem Katalysator aus Beispiel 4, wurden Flüsse von 27,5 g/h Wasserstoffperoxid (20 Gew.-%), 65 g/h Methanol und 13,7 g/h Propen bei 40 °C Reaktionstemperatur und 20 bar Reaktionsdruck durchgeleitet. Nach Verlassen des zweiten Reaktors wurde die Reaktionsmischung in einen Sambay-Verdampfer gegen Atmosphärendruck entspannt. Die abgetrennten Leichtsieder wurden on-line in einem Gaschromatographen analysiert. Der flüssige Reaktionsaustrag wurde gesammelt, gewogen und ebenfalls gaschromatographisch analysiert.
Während der gesamten Laufzeit sank der Wasserstoffperoxid-Umsatz von ursprünglich 98 % und erreichte nach 250 h einen Wert von 60 %. Die Selektivität von Propylenoxid bezüglich Wasserstoffperoxid betrug über die Laufzeit 95 % .

### Beispiel 6

Der deaktivierte Katalysator aus Beispiel 5 wurde in ein Quarzglasrohr eingebaut. Der deaktivierte Katalysator wurde daraufhin in einem Röhrenofen bei einer Heizrate von 4 °C/min bei einem Gasstrom aus 20 l Stickstoff/h auf 450 °C erhitzt. Danach wurden in den nächsten zwei Stunden der Sauerstoffgehalt im Inertgas auf 9 Vol.- % gesteigert und dort gehalten. Anschließend wurde in den nächsten 14 h der Sauerstoffgehalt auf 18 Vol.-% gesteigert und dort gehalten. Danach wurde der regenerierte Katalysator unter Inertgas abgekühlt, ausgebaut und wieder zur Epoxidation verwendet.

### Beispiel 7

Durch eine Reaktorkaskade von zwei Reaktoren mit je 190 ml Reaktionsvolumen, gefüllt mit je 10 g regeneriertem Katalysator aus Beispiel 6, wurden Flüsse von 27,5 g/h Wasserstoffperoxid (20 Gew.-%), 65 g/h Methanol und 13,7 g/h Propylen bei 40 °C Reaktionstemperatur und 20 bar Reaktionsdruck durchgeleitet. Nach Verlassen des zweiten Reaktors wurde die Reaktionsmischung in einen Sambay-Verdampfer gegen Atmosphärendruck entspannt. Die abgetrennten Leichtsieder wurden on-line in einem Gaschromatographen analysiert. Der flüssige Reaktionsaustrag wurde gesammelt, gewogen und ebenfalls gaschromatographisch analysiert.
Während der gesamten Laufzeit sank der Wasserstoffperoxid-Umsatz von ursprünglich 98 % und erreichte nach 250 h einen Wert von ungefähr 60 %. Die Selektivität von Propylenoxid bezüglich Wasserstoffperoxid betrug über die Laufzeit 95 %.

Die angeführten Beispiele zeigen, daß durch die erfindungsgemäße Regenerierung der deaktivierten Katalysatoren die katalytische Aktivität der Katalysatoren ohne Einbußen wiederhergestellt werden kann.

## Patentansprüche

1. Verfahren zur Regenerierung eines Zeolith-Katalysators, das die folgenden Stufen (I) und (II) umfaßt:
(I) Aufheizen eines zumindest teilweise durch meist organische Beläge deaktivierten Katalysators auf eine Temperatur im Bereich 250 °C bis 600 °C in einer Atmosphäre, die weniger als 0,2 Vol.-% Sauerstoff enthält, und
(II) Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von 0,1 bis 4 Vol.-% aufweist, wobei dieser Gasstrom einen höheren Gehalt an Sauerstoff enthält als die Atmosphäre in Stufe (I),
wobei der Zeolith-Katalysator vor der Regenerierung bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren eingesetzt wurde,
und wobei der zumindest teilweise deaktivierte Katalysator vor dem Aufheizen gemäß Stufe (I) mit einem Lösungsmittel gewaschen wird, das ausgewählt wird aus der Gruppe bestehend aus Wasser, einem Alkohol, einem Aldehyd, einem Keton, einem Ether, einer Säure, einem Ester, einem Nitril, einem Kohlenwasserstoff sowie einem Gemisch aus zwei oder mehr davon, und das schon bei der Epoxidation von organischen Verbindungen mit mindestens einer C-C-Doppelbindung, zur Hydroxylierung von aromatischen organischen Verbindungen oder zur Umwandlung von Alkanen zu Alkoholen, Aldehyden und Säuren als Lösungsmittel fungiert, und das Waschen so durchgeführt wird, dass die jeweils am Katalysator anhaftenden Wertprodukte von diesem entfernt werden können, und Temperatur und Druck nicht so hoch gewählt werden, dass die meist organischen Beläge entfernt werden.

2. Verfahren nach Anspruch 1, das zusätzlich die folgende Stufe (III) umfaßt:
(III) Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von mehr als 4 bis 100 Vol.-% aufweist.

3. Verfahren nach Anspruch 1 oder 2, wobei das Aufheizen gemäß Stufe (I) mit einer Aufheizrate von 0,1 °C/min. bis 20 °C/min., vorzugsweise von 0,3 °C/min. bis 15 °C/min., und insbesondere von 0,5 °C/min. bis 10 °C/min durchgeführt wird.

4. Verfahren nach einem der Ansprüche 1 bis 3, das zusätzlich die folgende Stufe (IV) umfaßt:
(IV) Abkühlen des in Stufe (III) erhaltenen regenerierten Katalysators in einem Inertgasstrom, der bis zu 20 Vol.-% eines Flüssigkeitsdampfes, ausgewählt aus der Gruppe bestehend aus Wasser, einem Alkohol, einem Aldehyd, einem Keton, einem Ether, einer Säure, einem Ester, einem Nitril, einem Kohlenwasserstoff sowie einem Gemisch aus zwei oder mehr davon, enthalten kann.

5. Verfahren nach einem der vorstehenden Ansprüche, wobei der zumindest teilweie deaktivierte Katalysator nach dem Aufheizen gemäß Stufe (I) und vor der Beaufschlagung gemäß Stufe (II) bei einer Temperatur von 250 bis 800 °C gehalten wird.

6. Verfahren nach einem der vorstehenden Ansprüche, wobei die Sauerstoff-liefemde Substanz ausgewählt wird aus der Gruppe bestehend aus einem Stickoxid der Formel NₓO_{y}, wobei x und y so gewählt werden, daß sich ein neutrales Stickoxid ergibt, N₂O, einem N₂O-haltigen Abgasstrom aus einer Adipinsäure-Anlage, NO, NO₂, Ozon, einem Gemisch aus zwei oder mehr davon.

7. Verfahren nach einem der Ansprüche 1 bis 5, wobei die Sauerstoff-liefemde Substanz CO₂ ist und die Stufen (II) und (III) bei einer Temperatur im Bereich von 500 bis 800 °C durchgeführt werden.

8. Verfahren nach einem der vorstehenden Ansprüche, wobei der Zeolith-Katalysator ausgewählt wird aus der Gruppe bestehend aus einem Titan-, Zirkonium-, Vanadium-, Chrom- oder Niob- enthaltenden Silikalit, mit MFI-, BEA-, MOR-, TON-, MTW-, FER-, CHA-, ERI-, RHO-, GIS-, BOG-, NON-, EMT-, HEU-, KFI-, TAU-, DDR-, MTT-, RUT-, LTL-, MAZ-, GME-, NES-, OFF-SGT-, EUO-, MFS-, MCM-22-, MEL-Struktur, MFI/MEL-Mischstruktur und einem Gemisch aus zwei oder mehr davon.

## Claims

1. A process for regenerating a zeolite catalyst, which comprises the following stages (I) and (II):
(I) heating a catalyst which is partially or completely deactivated by mostly organic coating to a temperature in the range of 250°C to 600°C in an atmosphere which contains less than 0.2% by volume of oxygen; and
(II) treating the catalyst at a temperature in the range from 250 to 800 °C, preferably 350 to 600 °C, with a gas stream which contains from 0.1 to 4% by volume of an oxygen-donating substance or of oxygen or of a mixture of two or more thereof, this gas stream containing more oxygen than the atmosphere in stage (I),
wherein the zeolite catalyst before the regeneration was employed in the epoxidation of organic compounds having at least one C-C double bond, for the hydroxylation of aromatic organic compounds or for the conversion of alkanes into alcohols, aldehydes and acids, and
wherein the partially or completely deactivated catalyst is washed, before the heating according to stage (I), with a solvent selected from the group consisting of water, an alcohol, an aldehyde, a ketone, an ether, an acid, an ester, a nitrile, a hydrocarbon, and a mixture of two or more thereof, which solvent is used as solvent for the epoxidation of organic compounds having at least one C-C double bond, for the hydroxylation of aromatic organic compounds or for the conversion of alkanes into alcohols, aldehydes and acids, and
wherein the washing is carried out in such a way that the particular desired products adhering to the catalyst can be removed therefrom but the temperature and pressure are not chosen so high that the mostly organic coating are also removed.

2. A process as claimed in claim 1, which additionally comprises the following stage (III):
(III) treating the catalyst at a temperature in the range from 250 to 800 °C, preferably from 350 to 600 °C, with a gas stream which contains from more than 4 to 100% by volume of an oxygen-donating substance or of oxygen or of a mixture of two or more thereof.

3. A process as claimed in claim 1 or 2, wherein the heating according to stage (I) is carried out at a heating rate of from 0.1 to 20 °C/min, preferably from from 0.3 to 15 °C/min, in particular from 0.5 to 10 °C/min.

4. A process as claimed in any of claims 1 to 3, which additionally comprises the following stage (IV):
(IV) cooling of the regenerated catalyst obtained in stage (III) in an inert gas stream which may contain up to 20% by volume of a vaporized liquid selected from the group consisting of water, an alcohol, an aldehyde, a ketone, an ether, an acid, an ester, a nitrile, a hydrocarbon, and a mixture of two or more thereof.

5. A process as claimed in any of the preceding claims, wherein the partially or completely deactivated catalyst is kept at a temperature from 250 to 800 °C after the heating according to stage (I) and before treatment according to stage (II).

6. A process as claimed in any of the preceding claims, wherein the oxygen-donating substance is selected from the group consisting of an oxide of nitrogen of the formula NₓO_{y}, where x and y are chosen to give a neutral oxide of nitrogen, N₂O, an N₂O containing exit gas stream from an adipic acid plant, NO, NO₂, ozone, and a mixture of two or more thereof.

7. A process as claimed in any of claims 1 to 5, wherein the oxygen-donating substance is CO₂ and stages (II) and (III) are carried out at a temperature in the range of from 500 to 800 °C.

8. A process as claimed in any of the preceding claims, wherein the zeolite catalyst is selected from the group consisting of a titanium-, zirconium-, vanadium-, chromium- and niobium-containing silicalite, having the MFI, BEA, MOR, TON, MTW, FER, CHA, ERI, RHO, GIS, BOG, NON, EMT, HEU, KFI, FAU, DDR, MTT, RUT, LTL, MAZ, GME, NES, OFF, SGT, EUO, MFS, MCM-22, MEL structure, mixed MFI/MEL structure and a mixture of two or more thereof.

## Revendications

1. Procédé de régénération d'un catalyseur zéolitique qui comprend les étapes (I) et (II) suivantes :
(I) un chauffage d'un catalyseur au moins partiellement désactivé par les dépôts en majeure partie organiques à une température de l'ordre de 250°C à 600°C dans une atmosphère qui contient moins de 0,2% en volume d'oxygène, et
(II) une alimentation du catalyseur, à une température de l'ordre de 250 à 800°C, de préférence de 350 à 600°C, en un courant gazeux qui présente une teneur en une substance fournissant de l'oxygène ou en oxygène ou en un mélange de deux ou de plusieurs de ceux-ci de l'ordre de 0,1 à 4% en volume, ce courant gazeux contenant une teneur en oxygène supérieure à l'atmosphère de l'étape (I),
dans lequel le catalyseur zéolitique avant la régénération est mis en oeuvre pour l'époxydation de composés organiques comportant au moins une double liaison C-C, pour l'hydroxylation de composés organiques aromatiques ou pour la conversion d'alcanes en alcools, aldéhydes et acides, et
dans lequel le catalyseur au moins partiellement désactivé est, avant le chauffage selon l'étape (I), lavé par un solvant qui est choisi parmi le groupe constitué d'eau, d'un alcool, d'un aldéhyde, d'une cétone, d'un éther, d'un acide, d'un ester, d'un nitrile, d'un hydrocarbure ainsi que d'un mélange de deux ou de plusieurs de ces substances, et qui fait déjà office de solvant lors de l'époxydation de composés organiques comportant au moins une double liaison C-C, lors de l'hydroxylation de composés organiques aromatiques ou lors de la conversion d'alcanes en alcools, aldéhydes et acides,
et le lavage est réalisé de manière que, si les produits cibles adhérant encore respectivement au catalyseur peuvent être éliminés, la température et la pression ne soient pas choisies suffisamment élevées pour éliminer aussi les dépôts en majeure partie organiques.

2. Procédé suivant la revendication 1, **caractérisé en ce qu'**il comprend en supplément l'étape (III) suivante :
(III) une alimentation du catalyseur, à une température de l'ordre de 250 à 800°C, de préférence de 350 à 600°C, en un courant gazeux qui présente une teneur en une substance fournissant de l'oxygène ou en oxygène ou en un mélange de deux ou de plusieurs de ceux-ci de l'ordre de plus de 4 à 100% en volume.

3. Procédé suivant l'une des revendications 1 et 2, dans lequel le chauffage suivant l'étape (I) est effectué à une vitesse d'échauffement de 0,1°C/minute à 20°C/minute, de préférence de 03°C/minute à 15°C/minute, et en particulier de 0,5°C/minute à 10°C/minute.

4. Procédé suivant l'une des revendications 1 à 3, **caractérisé en ce qu'**il comprend en supplément l'étape (IV) suivante :
(IV) un refroidissement du catalyseur régénéré obtenu dans l'étape (III) dans un courant gazeux inerte qui peut contenir jusqu'à 20% en volume d'une vapeur de liquide, choisi parmi le groupe constitué d'eau, d'un alcool, d'un aldéhyde, d'une cétone, d'un éther, d'un acide, d'un ester, d'un nitrile, d'un hydrocarbure ainsi que d'un mélange de deux ou de plusieurs de ces substances.

5. Procédé suivant l'une des revendications précédentes, dans lequel le catalyseur au moins partiellement désactivé après le chauffage selon l'étape (I) et avant l'alimentation selon l'étape (II) est maintenu à une température de 250 à 800°C.

6. Procédé suivant l'une des revendications précédentes, dans lequel la substance fournissant de l'oxygène est choisie parmi le groupe constitué d'un oxyde d'azote de la formule NₓO_{y}, où x et y sont choisis de façon à obtenir un oxyde d'azote neutre, de N₂O, d'un courant de gaz résiduaire contenant N₂O et provenant d'une installation d'acide adipique, de NO, de NO₂, d'ozone, et d'un mélange de deux ou de plusieurs de ces substances.

7. Procédé suivant l'une des revendications 1 à 5, dans lequel la substance fournissant de l'oxygène est CO₂ et en ce que les étapes (II) et (III) sont effectuées à une température de l'ordre de 500 à 800°C.

8. Procédé suivant l'une des revendications précédentes, dans lequel le catalyseur zéolitique est choisi parmi le groupe constitué d'un silicalite contenant du titane, du zirconium, du vanadium, du chrome ou du niobium avec une structure MFI, BEA, MOR, TON, MTW, FER, CHA, ERI, RHO, GIS, BOG, NON, EMT, HEU, KFI, FAU, DDR, MTT, RUT, LTL, MAZ, GME, NES, OFF, SGT, EUO, MFS, MCM-22, MEL, une structure mixte MFI/MEL, et d'un mélange de deux ou de plusieurs de ces substances.
